# EUROPEAN PATENT SPECIFICATION

(11) **EP 2 255 814 B1**
(45) Date of publication and mention of the grant of the patent: **08.04.2015**
(21) Application number: 09717725.7
(22) Date of filing: 27.02.2009
(51) Int. Cl.: A61K 31/683, A23L 1/30, A61K 8/55, A61K 31/685, A61P 17/00, A61P 43/00, A61Q 19/08

(54) **AGENT FOR INCREASING THE QUANTITY OF HYALURONIC ACID**
MITTEL ZUR STEIGERUNG DER HYALURONSÄUREMENGE
AGENT DESTINÉ À FAIRE AUGMENTER LA QUANTITÉ D'ACIDE HYALURONIQUE

(30) Priority: 04.03.2008 JP 2008053757
(43) Date of publication of application: 01.12.2010
(73) Proprietor: Nagase ChemteX Corporation, Osaka-shi, Osaka 550-8668 (JP)
(72) Inventor: SHIOJIRI, Masatoshi, Kobe-shi Hyogo 651-2241 (JP); SHIIHARA, Misa, Kobe-shi Hyogo 651-2241 (JP)
(74) Representative: Teipel, Stephan
(86) International application number: PCT/JP2009/000905
(87) International publication number: WO 2009/110205

(56) References cited:
- WO-A1-2006/043788
- WO-A1-2006/128465
- WO-A1-2009/028220
- JP-A- 2000 086 480
- JP-A- 2002 029 956
- JP-A- 2004 269 433
- JP-A- 2005 053 879
- JP-A- 2006 193 427
- JP-T- 2005 519 995
- US-A1- 2005 232 953
- TRISCOTT, M.X. ET AL.: 'Solubilization of hyaluronic acid synthetic activity from streptococci and its activation with phospholipids' J BIOL CHEM vol. 261, no. 13, 1986, pages 6004 - 6009, XP008140934
- FUMIO YAMAUCHI: 'Series <Shokuhin no Kagaku>' DAIZU NO KAGAKU (5TH PRINT) 1997, page 47

## Description

### TECHNICAL FIELD

The present invention relates to a hyaluronan-increasing agent and a skin elasticity improving agent.

### BACKGROUND ART

The skin consists of the epidermis and the underlying layer dermis. In the dermis, unlike epidermis, cells are sparsely-arranged and matrix fills in the void between the cells. This matrix is constituted by acid mucopolysaccharides such as hyaluronan produced by fibroblasts, and fibrous proteins such as collagen and elastin. It is known that hyaluronan, which is present only in an extremely small amount in the extracellular matrix in the dermis, is one of the most responsible components for skin elasticity. Since hyaluronan can retain as much as 5 to 6 L of water per gram, the water-holding capacity of the skin is very largely attributed to hyaluronan. Hyaluronan with such a high water-holding capacity incorporates a large amount of water to form a hydrated gel and thereby can keep the swelling pressure high in the extracellular matrix. This swelling pressure in the dermis leads to skin elasticity.

The level of hyaluronan in the skin is at peak during the fetal period, and decreases with age. When the level of hyaluronan in infants is set to 100%, the level in adult and elderly people is as low as 25 to 50% of the level in infants. In addition, the metabolic turnover rate of hyaluronan is high and its half-life in the adult skin is as short as not more than one day. A decline in hyaluronan production due to aging and metabolism makes the skin lose the water-holding capacity and elasticity. In order to ameliorate such skin troubles, a method for providing the skin with hyaluronan, and a method for promoting production of hyaluronan from skin cells such as fibroblasts are proposed.

However, since hyaluronan is hard to absorb into the skin, methods for providing the skin with hyaluronan do not produce sufficient effects.

Examples of hyaluronan production promoters include an asparagus extract and a butcherbroom extract both disclosed in patent literature 1, a plant extract disclosed in patent literature 2, and plant extracts disclosed in patent literatures 3, 4 and 5. However, since most of these extracts have a high polarity and a low availability in the skin, they cannot necessarily provide satisfying effects.

Meanwhile, phospholipid mainly including phosphatidylcholine, phosphatidylethanolamine, phosphatidylinositol and phosphatidylserine, which is a constituent of the biological membrane, is conventionally used as a microcapsule (liposome) for cosmetics because of its amphiphilicity and high safety (patent literature 6). Also, phospholipid has been known to enhance permeation of drugs into the skin (patent literature 7).

Patent literature 8 discloses a composition comprising phosphatidylserine, one kind of phospholipid, as an active ingredient for skin protection or improvement, enhancement of barrier functions, inhibition or alleviation of inflammatory reactions, treatment or improvement of atopic skin, etc. In the literature, it is confirmed that the phosphatidylserine-containing composition activates PPARα in skin cells and thereby protects the skin from damage caused by outer stimuli such as ultraviolet and chemicals, or improves such skin damage. This literature also discloses that phosphatidylserine decreases the expression of the collagenase MMP-1 in fibroblasts and promotes production of collagen precursors.

Patent literature 9 discloses a composition comprising one or more kinds selected from saccharides, maltitol, sulfated saccharides or salts thereof, saccharide alkyl ethers and acylated saccharides; a phospholipid; and a carboxyvinyl alkyl crosspolymer, and teaches that the composition can be used as makeup cosmetics for rough and sensitive skin.

Triscott et al J. Biol chem. 1986; 261(13): 6004-6009 found that phosphatidyl serine and phosphatidyl inositol improve the synthesis of hyaluronic acid in vitro.

### [Citation List]

Patent Literature 1: JP-A 2007-262012
Patent Literature 2: JP-A 2007-084448
Patent Literature 3: Japanese Patent No. 4022911
Patent Literature 4: Japanese Patent No. 3998085
Patent Literature 5: Japanese Patent No. 3522609
Patent Literature 6: Japanese Patent No. 3521517
Patent Literature 7: Japanese Patent No. 2714413
Patent Literature 8: JP-W 2007-522259
Patent Literature 9: Japanese Patent No. 3659793

### DISCLOSURE OF THE INVENTION

### PROBLEM TO BE SOLVED BY THE INVENTION

An object of the present invention is to provide a hyaluronan-increasing agent comprising a natural active ingredient, and a skin elasticity improving agent comprising the same.

### MEANS FOR SOLVING THE PROBLEM

The present inventors conducted extensive research to achieve the above-mentioned object. As a result, the inventors obtained the following findings.
(i) Percutaneous, oral or any other administration of a glycerophospholipid comprising phosphatidylserine (hereinafter sometimes abbreviated as "PS") and phosphatidylinositol (hereinafter sometimes abbreviated as "PI") increases the production of hyaluronan in the skin and inhibits the degradation of hyaluronan therein, resulting in increase in the level of hyaluronan in the skin.
(ii) Percutaneous, oral or any other administration of the mixture of PS and PI remarkably increases skin viscoelasticity.

The present invention was completed based on these findings, and provides the following hyaluronan-increasing agents, hyaluronan production promoters, hyaluronan degradation inhibitors, skin elasticity improving agents, food compositions, etc.
1. A non-therapeutic method for increasing the level of hyaluronan in the skin, said method comprising administering, to a human, an effective amount of phosphatidylserine and phosphatidylinositol.
2. The method according to above 1, wherein the weight ratio of phosphatidylserine (PS) to phosphatidylinositol (PI) (PS:PI) administered to the human is 0.5 to 20:1 in terms of dry weight.
3. The method according to above 1 or 2, wherein the phosphatidylserine and phosphatidylinositol are externally administered to the skin of the human.
4. The method according to above 1 or 2, wherein the phosphatidylserine and phosphatidylinositol are orally administered to the human.
5. Phosphatidylserine and phosphatidylinositol for use in the treatment of a disease wherein hyaluronan level is decreased.
6. The phosphatidylserine and phosphatidylinositol according to above 5, wherein the weight ratio of phosphatidylserine (PS) to phosphatidylinositol (PI) (PS:PI) is 0.5 to 20:1 in terms of dry weight.
7. The phosphatidylserine and phosphatidylinositol according to above 5 or 6, which is for external use.
8. The phosphatidylserine and phosphatidylinositol according to above 5 or 6, which is for oral use.
9. The phosphatidylserine and phosphatidylinositol according to any of above 5-8, wherein the disease wherein hyaluronan level is decreased involves deficiency of hyaluronan synthase or decreased expression thereof.
10. A non-therapeutic method for improving skin elasticity which comprises orally administering an effective amount of phosphatidylserine and phosphatidylinositol to a human.
11. The method according to above 10, wherein the weight ratio of phosphatidylserine (PS) to phosphatidylinositol (PI) (PS:PI) is 0.5 to 20:1 in terms of dry weight.

### EFFECT OF THE INVENTION

Hyaluronan is abundantly present in the joint, vitreous body, skin, brain, etc. in the living body. Although hyaluronan easily degrades and has a short half-life, administration of the glycerophospholipids PS and PI via oral, percutaneous or any other route inhibits degradation of hyaluronan by hyaluronidase etc. in the living body. The glycerophospholipid also increases the level of hyaluronan synthase, and as a result, increases the production of hyaluronan. Consequently, the glycerophospholipid brings increase in the level of hyaluronan in the living body (typically in the joint, vitreous body, skin, brain, etc.). Particularly, combined use of PI and PS increases the level of hyaluronan significantly and improves skin elasticity.

The glycerophospholipid, which is derived from natural materials such as soybeans, can be said to be safe and prepared by a nature-friendly method.

### BRIEF DESCRIPTION OF DRAWINGS

Fig. 1 is a graph showing that the expression of hyaluronan synthase is increased by contact of human fibrocytes with the glycerophospholipid of the present invention.
Fig. 2 is a graph showing that the level of hyaluronan is increased by contact of human fibrocytes with the glycerophospholipid of the present invention.
Fig. 3 is a graph showing that the level of hyaluronan is increased by contact of the skin model with the glycerophospholipid of the present invention.
Fig. 4 is a graph showing that skin viscoelasticity is increased by dermal application of the glycerophospholipid of the present invention.
Fig. 5 is a graph showing that skin viscoelasticity is increased by oral administration of the glycerophospholipid of the present invention.

### BEST MODE FOR CARRYING OUT THE INVENTION

Hereinafter, the present invention will be illustrated in detail.

### (I) Hyaluronan-increasing agent

The hyaluronan-increasing agent for use in the present invention comprises, as active ingredients the glycerophospholipids PS and PI (hereinafter sometimes referred to as "the glycerophospholipid of the present invention").

As mentioned above, the glycerophospholipid of the present invention increases the level of hyaluronan synthase, inhibits degradation of hyaluronan by hyaluronidase, and thereby increases the level of hyaluronan in the living body. For this reason, the glycerophospholipid of the present invention can be used as an active ingredient of a hyaluronan production promoter or a hyaluronan degradation inhibitor.

### (1) The glycerophospholipid of the present invention

The weight ratio of PS to PI (PS:PI) is preferably about 0.5 to 20:1, more preferably about 1 to 10:1, and even more preferably about 1 to 4:1 in terms of dry weight.

PS is commercially available from SIGMA, BIOMOL, NOF CORPORATION, etc. PI is commercially available from BIOMOL etc. The mixture of PS and PI can be prepared by mixing these commercial products.

Alternatively, PI can be obtained by isolation from lecithin derived from vegetables such as soybeans, rapeseeds, sunflowers and palms. PI can be also isolated from commercial lecithin such as SLP-WHITE (phosphatidylcholine (hereinafter sometimes referred to as "PC") 25.6%, phosphatidylethanolamine (hereinafter sometimes referred to as "PE") 24.1%, phosphatidic acid (hereinafter sometimes referred to as "PA") 8%, PI 12%, others about 30%), and SLP-PI powder (PC 18%, PE 22%, PA 8%, PI 17%, others about 35%)(both are manufactured by Tsuji Oil Mill co., Ltd.). PI isolation can be performed using various kinds of chromatography.

The above-mentioned vegetable lecithin and commercial lecithin contain PC and PE. A glycerophospholipid mixture containing PS can be obtained by allowing serine and phospholipase D (hereinafter sometimes referred to as "PLD") to act on such lecithin in base exchange reaction. Further, from this mixture, PS can be isolated by use of various kinds of chromatography. PI or PS may be formulated into preparations without removal of foreign substances such as other glycerophospholipids.

The above-mentioned vegetable lecithin and commercial lecithin contain PI, PC and PE. A glycerophospholipid mixture containing PS and PI can be obtained by allowing serine and PLD to act on such lecithin in base exchange reaction. From this mixture, a mixture of PS and PI is isolated if needed. The mixture of PI and PS may be formulated into preparations without removal of foreign substances such as other glycerophospholipids.

A glycerophospholipid mixture in which the weight ratio of PS to PI (dry weight ratio; PS:PI) is about 0.5 to 20:1 can be obtained by the above-mentioned base exchange reaction using, as a starting material, lecithin in which the weight ratio of PI to the total of PC and PE (PI/(PC+PE)) is about 20 to 45% by weight, and more preferably about 25 to 40% by weight in terms of dry weight. The contents of PC, PE and PI in the above-mentioned vegetable lecithin and commercial lecithin are usually within this range, but when the content of the total of PC and PE is lower, the weight ratio (dry weight ratio) of PI to the total of PC and PE (PI/(PC+PE)) in the lecithin can be adjusted to fall within the above range by addition of highly pure phospholipid such as SLPC55 (manufactured by Tsuj i Oil Mill co., Ltd.).

The base exchange reaction of lecithin can be performed by the method described in JP-A 2007-014270, for example.

Namely, first, lecithin is stirred in a two-phase system consisting of an organic phase and an aqueous phase, before the base exchange reaction starts. The organic solvent is not particularly limited, and examples thereof include known organic solvents such as aliphatic hydrocarbons, cyclic aliphatic hydrocarbons, halogenated hydrocarbons, esters, ethers and ketones. The aqueous phase is preferably a buffer solution. The total concentration of an acid/base and a salt thereof in the buffer solution is preferably about 0.1 to 2 M. In the two-phase system, the ratio of water to the organic solvent is preferably 20% by weight or less. Stirring in the two-phase system can be performed at a temperature of about 10 to 40°C for about 30 minutes to 2 hours. Thus, usually, an emulsion of the glycerophospholipid, the organic solvent and water is formed.

Next, serine and PLD are added to the mixture. Here, the concentration of the glycerophospholipid in the base exchange reaction system is preferably about 5 to 40% by weight. The concentration of serine in this reaction system is preferably about 20 to 40% by weight.

The PLD is not particularly limited, and examples thereof include phospholipase D manufactured by Nagase ChemteX Corporation, Streptomyces chromofuscus-derived phospholipase D and Streptomyces species-derived phospholipase D both manufactured by Sigma, etc. The concentration of the PLD in this reaction system can be about 5 to 200 U per gram of the glycerophospholipid. The 1 U of PLD corresponds to the amount of the enzyme capable of releasing 1 µmol of choline per minute in the reaction with a substrate, 95% soybean phosphatidylcholine, in a 0.2 M acetate buffer solution (containing 10 mM CaCl₂ and 1.3% Triton X-100, pH 4.0) containing 0.16% of the substrate at 37°C.

The base exchange reaction can be performed at a pH of about 3.5 to 10 at a temperature of about 10 to 40°C for about 1 to 72 hours.

In the above-mentioned base exchange reaction, PC, PE, etc. in the glycerophospholipid and serine react to produce PS. In this base exchange reaction, PI hardly reacts with compounds containing a hydroxyl group, and for this reason, almost all PI in the starting material (glycerophospholipid) is contained as it is in the resultant (phospholipid mixture) after the base exchange reaction.

The base exchange reaction can be completed by heat inactivation of the PLD. After the organic phase is obtained by centrifugation etc., the organic solvent is evaporated in vacuo for concentration of the reaction mixture. Then, the residue is crystallized with acetone or ethanol, and the resulting solids are separated by solid-liquid separation and dried. Thus, a glycerophospholipid mixture containing PS and PI can be isolated.

### (2) Preparations

The hyaluronan-increasing agent for use in the present invention can be in the form of cosmetics, quasi drugs, pharmaceuticals, food compositions, etc.

### External preparations for skin

A typical example of the hyaluronan-increasing agent for use in the present invention is external preparations for skin. The form of the external preparation for skin is not particularly limited, and in the case of cosmetics, examples thereof include skin care and makeup cosmetics such as lotion, cosmetic milky lotion, cosmetic cream, cosmetic gel, essence, pack, foundation, lip stick, lip cream, lip gloss, face wash, body wash, hand cream, shampoo, rinse and hair styling products. Inter alia, cosmetic gel, cosmetic milky lotion and cosmetic cream are preferred because they can be applied to a wide region of the skin, and cosmetic gel is more preferred.

These cosmetics can be prepared by blending the glycerophospholipid of the present invention with an ingredient usually used for cosmetics, for example, a base such as purified water, alcohols (lower alcohols, polyhydric alcohols, etc.), fats and oils, waxes and hydrocarbons, and optionally an additive such as surfactants, thickeners, ultraviolet absorbers, ultraviolet scattering agents, stabilizers, preservatives, colorants and fragrances.

In the case of pharmaceuticals or quasi drugs, exemplary forms of the external preparation for skin include ointments, gels, liniments, lotions, emulsions, powders, suspensions, aerosols, liquids as well as preparations having a base on the support, such as plasters, cataplasms and tapes. Inter alia, gels, lotions and emulsions are preferred because they can be uniformly applied to the skin without any feeling of greasiness, and gels are more preferred.

External preparations for skin as pharmaceuticals or quasi drugs can be prepared by blending the glycerophospholipid of the present invention into an appropriate base. Examples of the base include polymers such as sodium alginate, gelatin, cornstarch, tragacanth gum, methylcellulose, hydroxyethylcellulose, carboxymethylcellulose, xanthan gum, carrageenan, mannan, agarose, dextrin, carboxymethyl starch, polyvinyl alcohol, sodium polyacrylate, a methoxy ethylene-maleic anhydride copolymer, polyvinyl ether, polyvinyl pyrrolidone, a carboxyvinyl polymer, hydroxypropylcellulose, hydroxypropylmethylcellulose and pullulan; hydrocarbons such as white petrolatum, yellow petrolatum, paraffin, ceresin wax and microcrystalline wax; gel hydrocarbons (for example, trade name: Plastibase manufactured by Bristol-Myers Squibb); higher fatty acids such as stearic acid; higher alcohols such as cetanol, octyldodecanol and stearyl alcohol; polyethylene glycol (for example, macrogol 4000 etc.); polyhydric alcohols such as propylene glycol, glycerin, dipropylene glycol, 1,3-butylene glycol and concentrated glycerin; fatty acid esters such as monooleates and glyceryl stearate; and a phosphate buffer solution. Furthermore, an additive such as solubilizers, inorganic fillers, pH adjusters, moisturizers, preservatives, thickeners, antioxidants and refrigerants may be contained.

The content of the glycerophospholipid for use in the present invention in the external preparation for skin is as follows. The total content of PS and PI is preferably about 0.01 to 7.5% by weight, more preferably about 0.05 to 5% by weight and even more preferably about 0.1 to 2% by weight relative to the whole preparation in terms of dry weight. When the content is within the above-mentioned range, an increasing effect on the level of hyaluronan is sufficiently achieved by use of the external preparation for skin in a proper amount.

### Oral preparations

The hyaluronan-increasing agent for use in the present invention can be also formulated into various kinds of oral preparations. Examples thereof include powders, granules, tablets, pills, capsules, chewables as solid preparations; and emulsions, liquids and syrups as liquid preparations. Inter alia, granules, tablets and capsules are preferred because they are easy to take orally and palatable, and granules are more preferred.

The solid preparations can be prepared by blending a pharmaceutically acceptable carrier and additive with the active ingredient, i.e., the glycerophospholipid of the present invention. For example, an excipient such as sucrose, lactose, glucose, starch and mannite; a binder such as gum arabic, gelatin, crystalline cellulose, hydroxypropylcellulose and methylcellulose; a disintegrant such as carmellose and starch; a stabilizer such as anhydrous citric acid, sodium laurate and glycerol; and the like can be blended. In addition, the solid preparations may be coated with gelatin, sucrose, gum arabic, carnauba wax, etc., or encapsulated. The liquid preparations can be prepared by dissolving or dispersing the above-mentioned active ingredient in water, ethanol, glycerin, simple syrup or a mixture thereof. These preparations may contain an additive such as sweeteners, preservatives, demulcents, lubricants, diluents, buffering agents, aromatizing agents and colorants.

The content of the glycerophospholipid for use in the present invention in the oral preparation is as follows. The total content of PS and PI is preferably about 0.01 to 40% by weight, more preferably about 0.02 to 30% by weight and even more preferably about 0.05 to 20% by weight relative to the whole preparation in terms of dry weight. When the content is within the above-mentioned range, an increasing effect on the level of hyaluronan is sufficiently achieved by use of the oral preparation in a proper amount.

### Other preparations

In the case where the hyaluronan-increasing agent for use in the present invention is in the form of pharmaceuticals, it can be also formulated into injections, suppositories or the like.

The injections can be prepared by dissolving or dispersing the glycerophospholipid of the present invention in distilled water for injection, physiological saline or the like. In addition, a water-soluble inorganic acid or its salt, a water-soluble organic acid or its salt, a neutral amino acid, an acidic amino acid or their salts, a salt of a basic amino acid, etc. may be contained as a pH adjuster etc. A buffering agent, a stabilizer, a soothing agent, a preservative, etc. may be also contained.

The content of the glycerophospholipid for use in the present invention in the injection is as follows. The total content of PS and PI is preferably about 0.001 to 5% by weight, more preferably about 0.01 to 1% by weight and even more preferably about 0.05 to 0.5% by weight relative to the whole preparation in terms of dry weight. When the content is within the above-mentioned range, an increasing effect on the level of hyaluronan is sufficiently achieved by use of the injection in a proper amount.

The suppositories can be prepared by blending the glycerophospholipid for use in the present invention into a base such as acrylic polymers such as Carbopol and polycarbophil; cellulosic polymers such as hydroxypropylcellulose and hydroxypropylmethylcellulose; natural polymers such as sodium alginate and chitosan; fatty acid waxes; etc. In addition, a preservative such as sodium benzoate, potassium sorbate and paraben; a pH adjuster such as hydrochloric acid, citric acid and sodium hydroxide; and a stabilizer such as methionine may be blended.

The content of the glycerophospholipid for use in the present invention in the suppository is as follows. The total content of PS and PI is preferably about 0.02 to 5% by weight, more preferably about 0.05 to 3% by weight and even more preferably about 0.1 to 2% by weight relative to the whole preparation in terms of dry weight. When the content is within the above-mentioned range, an increasing effect on the level of hyaluronan is sufficiently achieved by use of the suppository in a proper amount.

### (3) Food compositions

In the case where the hyaluronan-increasing agent for use in the present invention is in the form of a food composition, the food composition contains about 0.01 to 40% by weight of the above-mentioned glycerophospholipid of the present invention in terms of dry weight. The content of the glycerophospholipid of the present invention in the food composition is preferably about 0.02 to 30% by weight, and more preferably about 0.05 to 20% by weight. The content of PS and PI is preferably about 0.01 to 30% by weight, and more preferably about 0.02 to 20% by weight. When the content is within the above-mentioned range, the glycerophospholipid in an amount sufficient to increase the hyaluronan level and improve skin elasticity will be contained in a reasonably consumable amount of a food.

Such a food composition is suitable for use as a health food (supplement) and is preferable for use as a food with health claims (a food for specified health use, a food with nutrient function claims).

By blending with an excipient or additive usually used for food, the food composition of the present invention can be formulated into tablets, pills, granules, powders, capsules, water-dispersible powders, emulsions, liquids, extracts, elixirs, etc. Inter alia, tablets and granules are preferred because they are easy to take orally and palatable, and granules are more preferred.

Examples of the excipient usually used for food include binders such as syrup, gum arabic, sucrose, lactose, powdered reduction maltose, cellulosic sugar, mannitol, maltitol, dextran, starch, gelatin, Sorbit, tragacanth and polyvinyl pyrrolidone; lubricants such as sucrose fatty acid esters, glycerol fatty acid esters, magnesium stearate, calcium stearate, talc and polyethylene glycol; disintegrants such as potato starch; and wetting agents such as sodium lauryl sulfate. Examples of the additive include fragrances, buffering agents, thickeners, colorants, stabilizers, emulsifiers, dispersants, suspending agents and preservatives.

Exemplary forms of the food composition may include drinks (sport drinks, drinkable preparations, milk drinks, lactic acid bacteria beverages, fruit-juice drinks, carbonated drinks, vegetable drinks, tea drinks, etc.) and confectionery (baked confectionery such as cookies, jelly, gum, gummy candies, candies, etc.). Inter alia, milk drinks are preferred because they are compatible with phospholipids and therefore easy to prepare.

### (4) Method for use

In the case where the hyaluronan-increasing agent for use in the present invention is in the form of pharmaceuticals, the dose of the hyaluronan-increasing agent of the present invention varies with the age and skin conditions of the subject, but is generally as follows. In the case of oral preparations, the total dose of PS and PI is preferably about 0.01 to 0.5 g daily in terms of dry weight.

In the case of injections, the total dose of PS and PI is preferably about 0.005 to 100 mg daily in terms of dry weight.

In the case of suppositories, the total dose of PS and PI is preferably about 0.005 to 100 mg daily in terms of dry weight.

In the case of external preparations for skin, the dose of the preparation is about 0.1 to 1 mL daily, which is the usual amount for use.

In the case where the hyaluronan-increasing agent for use in the present invention is in the form of food compositions, the recommended intake of the food composition varies with the body weight, skin conditions and the like of the subject, but the total intake of PS and PI is preferably about 0.1 to 1.5 g daily in terms of dry weight.

The subject is not particularly limited, but preferably a person whose hyaluronan level is decreased due to aging or diseases, or a person who wants to prevent the hyaluronan level from decreasing. Another suitable subject is a person with deficiency of a hyaluronan synthase, such as HAS1, HAS2 and HAS3, or with a decreased expression thereof.

The present invention also includes a method for increasing the hyaluronan level in a living body, specifically, a method for promoting the production of hyaluronan and a method for inhibiting the degradation thereof, and a method for improving skin elasticity, each of which comprises administering the above-mentioned effective amount of the glycerophospholipid of the present invention to a mammal, especially a human.

### (II) Skin elasticity improving agent

The skin elasticity improving agent for use in the present invention contains PI and PS as active ingredients. The skin elasticity improving agent can be in the form of cosmetics, pharmaceuticals, quasi drugs, food compositions, etc. The other constitutions of the skin elasticity improving agent are the same as those described in respect of the hyaluronan-increasing agent.

### EXAMPLES

Hereinafter, the present invention will be illustrated in more detail by examples, but is not limited thereto.

### Preparation Example 1 (preparation of a PI-PS containing phospholipid mixture)

7 g of soy lecithin (UltralecP (ADM); contents: PC = 24% by weight, PE = 17% by weight, PI = 14% by weight) was mixed with and dissolved in 70 mL of a mixed solvent of heptane and acetone (heptane: acetone = 4:1), to give a lecithin solution. Apart from this, an enzyme-containing serine solution in which 20 g of serine and 500 U of PLD (manufactured by Nagase ChemteX Corporation) were contained in 67 mL of a 1 M acetate buffer solution (pH 4.5) was prepared.

The enzyme-containing serine solution was added to the lecithin solution, and the mixture was stirred at 30°C for 5 hours. Then, to this, 75 mL of the mixed solvent of heptane and acetone and 20 g of sodium chloride were added, and the mixture was stirred for 1 hour. This mixture was allowed to stand and separate into two phases, i.e. an aqueous phase and an organic phase. The organic phase was collected and then a PI-PS containing organic solution was obtained. This organic solution contained 8 g of solids. The composition of the solids determined by HPLC under the conditions described below was 32% by weight of PS, 21% by weight of PI, 2% by weight of PC, 9% by weight of PE, and 13% by weight of PA.

### <HPLC conditions>

Column: Unisil Q NH2 manufactured by GL Sciences (4.6 mm I.D. × 250 mm)
Mobile phase: acetonitrile/methanol/50 mM ammonium dihydrogenphosphate = 1856/874/270
Flow rate: 1.3 mL/min
Detection: UV 205 nm

### Preparation Example 2 (preparation of PI-PS liposome)

The solids of Preparation Example 1 were dissolved in chloroform, and the solution was evaporated. The residue was dispersed in Milli-Q water and the dispersion liquid was sonicated in iced water, to give PI-PS liposomes.

### Reference Preparation Example 3 (preparation of PI liposome)

L-α-phosphatidylinositol Na provided by BIOMOL was dissolved in a mixed solvent of chloroform/methanol (volume ratio: 9/1), and the solution was evaporated. The residue was dispersed in Milli-Q water and the dispersion liquid was sonicated in iced water, to give PI liposomes.

### Reference Preparation Example 4 (preparation of PS liposome)

L-α-phosphatidylserine provided by SIGMA was dissolved in a mixed solvent of chloroform/methanol (volume ratio: 9/1), and the solution was evaporated. The residue was dispersed in Milli-Q water and the dispersion liquid was sonicated in iced water, to give PS liposomes.

### Preparation Example 5 (preparation of PI-PS containing phospholipid emulsified liquid)

An aqueous solution containing 5% (w/v) glycerin, 5% (v/v) ethanol, 0.1% (w/v) methylparaben and 0.05% (w/v) propylparaben was made to contain 0.5% of the solids prepared in Preparation Example 1, to give a uniform emulsified liquid.

### <Example 1>

### Analysis of augmentation effect on mRNA expression of hyaluronan synthase HAS2

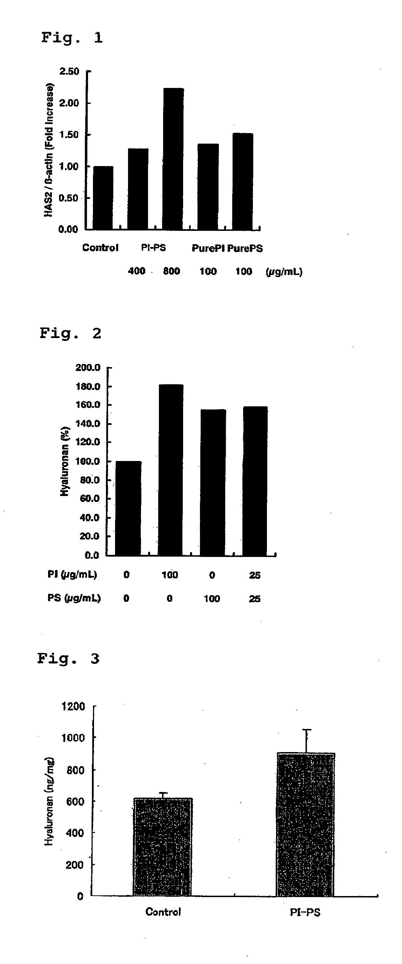

Normal human fibroblasts (NHDF(AD); Kurabo Industries Ltd.) were seeded at 8×10⁵/well in 6-well plates. After 24-hour preculture, the culture media were removed and replaced with culture media each containing a different phospholipid liposome prepared in Preparation Examples 2 to 4. The culture was maintained for 24 hours. The concentrations of PI and PS in the culture medium are as shown in Fig. 1. Then, after removal of the culture medium and addition of 1 mL of ISOGEN (NIPPON GENE), RNA was extracted from the cells according to the usual method described in the attachment of ISOGEN.

cDNA preparation was performed using SuperScriptIII First-Strand Synthesis System for RT-PCR (Invitrogen) according to the operating procedures described in the attachment of the kit. RT-PCR was performed using primers below.
HAS2 (Forward): GCCTCATCTGTGGAGATGGT (SEQ ID NO: 1)
HAS2 (Reverse): ATGCACTGAACACACCCAAA (SEQ ID NO: 2)
β-actin (Forward): GGACTTCGAGCAAGAGATGG (SEQ ID NO: 3)
β-actin (Reverse): AGCACTGTGTTGGCGTACAG (SEQ ID NO: 4)

Using each of the obtained cDNA and the HAS2 primer set, PCR was performed for 29 cycles of 94°C for 1 min, 58°C for 1 min, and 72°C for 1 min. Each of the obtained cDNA was also subjected to PCR for 21 cycles under the same conditions as above using the β-actin primer set. The DNA polymerase used was GoTaq (Promega). After completion of PCR, the PCR product was analyzed by electrophoresis in 1.5% agarose gel. Each band strength was determined by densitometry and the degree of increase in HAS2 mRNA expression was semi-quantitatively assessed by comparison of the HAS2/β-actin ratios between samples and the control.

The results are shown in Fig. 1. The vertical axis of Fig. 1 represents the ratio of the HAS2 mRNA level of cells made in contact with the glycerophospholipid relative to that of control cells not made in contact with the glycerophospholipid. The human fibroblast NHDF(AD) showed a significantly increased mRNA expression of HAS2 by contact with PI alone, PS alone and a glycerophospholipid mixture containing both of them.

### <Example 2>

### Study on hyaluronan production promoting effect

Normal human dermal fibroblasts (NHDF(AD); Kurabo Industries, Ltd.) were seeded at 2×10⁵/well in 24-well plates. After 24-hour preculture, the culture media were removed and replaced with culture media each containing a different phospholipid liposome at 100 µg/mL, i.e., the PI-PS containing liposome obtained in Preparation Example 2, the PI liposome obtained in Reference Preparation Example 3, or the PS liposome obtained in Reference Preparation Example 4. The culture was maintained for 24 hours. The culture supernatant was collected and hyaluronan therein was quantified by the ELISA method.

A hyaluronan binding protein (HABP; SEIKAGAKU CORPORATION) was added at 2 µg/mL to 96-well plates, which were then allowed to stand at 4°C for 24 hours. After this, the wells were washed. Blocking was performed at 4°C for 24 hours using Block Ace (Dainippon Sumitomo Pharma Co., Ltd.). After the wells were washed, a hyaluronan standard (Wako Pure Chemical Industries, Ltd.) or the culture supernatant was added to the wells and 1-hour incubation was performed at 37°C. After the wells were washed, biotinylated HABP (SEIKAGAKU CORPORATION) was added to the wells and 2-hour incubation was performed at 37°C. Avidin alkaline phosphatase conjugate (Wako Pure Chemical Industries, Ltd.) was added to the wells and 1-hour incubation was performed at 37°C. After the wells were washed, p-nitrophenylphosphate (Wako Pure Chemical Industries, Ltd.) was added to the wells. After the reaction at 37°C for 30 minutes, the absorbance at 405 nm was measured.

The results are shown in Fig. 2. The human dermal fibroblasts showed a significantly increased production of hyaluronan by contact with PI alone, PS alone and PI and PS. Particularly, PI and PS even in small amounts showed a high production of hyaluronan.

### <Example 3>

### Study on hyaluronidase inhibiting effect

Two different sample solutions were prepared by dispersing the PI-PS containing phospholipid mixture of Preparation Example 1 in an acetate buffer solution (0.1 M, pH 4) containing 1% DMSO: solutions containing the phospholipid mixture at 100 and 200 µg/ml. An enzyme solution was prepared by dissolving bovine testis hyaluronidase (TypeIV-S, Sigma) at 2 mg/ml in a 0.1 M acetate buffer solution (pH 4). An enzyme activation solution was prepared by dissolving Compound 48/80 (histamine releaser; a condensate of N-methyl-p-methoxyphenethylamine and formaldehyde; Sigma) at 0.5 mg/ml in a 0.1 M acetate buffer solution (pH 4) containing 3.8 mg/ml CaCl₂. A substrate solution was prepared by dissolving sodium hyaluronate (derived from crest) at 4 mg/ml in a 0.1 M acetate buffer solution (pH 4). A boric acid solution (pH 9.1) was prepared by dissolving 4.95 g of boric acid in 100 ml of water. A p-DAB reagent was prepared by dissolving 5 g of p-dimethylaminobenzaldehyde in 44 ml of acetic acid and 6 ml of 10 N HCl, and before use, diluting the resulting solution 10 times in acetic acid.

After 0.1 ml of each sample solution was incubated with 0.05 ml of the enzyme solution at 37°C for 20 minutes, 0.1 ml of the enzyme activation solution was added thereto and the mixture was incubated at 37°C for 20 minutes. After addition of 0.25 ml of the substrate solution, the enzyme reaction was performed at 37°C for 40 minutes. After addition of 0.1 ml of 0.4 N NaOH and 0.1 ml of the boric acid solution, the mixture was boiled for 3 minutes and then cooled rapidly. 3 ml of the p-DAB reagent was added to the mixture, and after incubation at 37°C for 20 minutes, the absorbance (585 nm) was measured. The absorbance at 585 nm is proportional to the concentration of the degradation product of sodium hyaluronate.

When the absorbance of the control (an acetic acid buffer solution (0.1 M, pH 4) containing 1% DMSO) was set to 100%, the relative values for sample solutions containing the PI-PS containing phospholipid mixture at 100 and 200 µg/ml were 50.8% and 12.2%, respectively. The PI-PS containing phospholipid mixture inhibited hyaluronidase in a dose-dependent manner, and thereby reduced the amount of the degradation product of sodium hyaluronate.

### <Example 4>

### Study on hyaluronan production promoting/degradation inhibiting effects using a skin model

By use of 3D normal skin model TESTSKIN LSE-high (Toyobo Co., Ltd.) that mimics the human skin conditions, the effect of the PI-PS containing phospholipid mixture on the level of hyaluronan in the skin model was assessed.

Specifically, a sample solution was prepared by dispersing the PI-PS containing phospholipid mixture at 5 mg/ml in a 10 mM citrate buffer solution (pH 5.5). A 10 mM citrate buffer solution (pH 5.5) was used as a control solution. After 50 µl of the sample solution was added to each well of TESTSKIN LSE-high, incubation was performed at 37°C in a CO₂ incubator for 24 hours. 24 hours later, the skin model was stripped and dried under reduced pressure at 60°C for 2 hours. After 1 ml of a 10 mM Tris-HCl buffer solution (pH 8.0) containing 2.5% actinase E (Kaken Pharma Co., Ltd.) was added to the stripped skin model, the enzyme reaction was performed at 55°C for 24 hours. 24 hours later, heat-treatment was performed at 100°C for 10 minutes. Then, after centrifugation at 12,000 rpm for 10 minutes, hyaluronan in the resulting supernatant was quantified by the ELISA method under the same conditions as those of Example 2.

The results are shown in Fig. 3. In the Fig. 3, PI-PS represents the sample solution containing the PI-PS containing phospholipid mixture. The level of hyaluronan in the skin tissue model treated with the control solution was 620 ng/mg (dry tissue weight), while the level of hyaluronan in the skin tissue model treated with the sample solution containing the PI-PS containing phospholipid mixture was 910 ng/mg (dry tissue weight). Thus, the PI-PS containing phospholipid mixture increased the level of hyaluronan in the skin model.

### <Example 5>

### Skin monitor test using external preparations for skin in humans

Each of subjects in their 30s to 40s (2 males and 2 females) was instructed to apply about 0.5 mL of the 0.5% PI-PS containing phospholipid emulsified liquid prepared in Preparation Example 5 onto the back of his/her right hand, and the equal volume of a control liquid prepared in the same manner as in Preparation Example 5 except that glycerophospholipid was not added, onto the back of his/her left hand, twice a day, i.e., every morning and evening, for 4 weeks. The skin viscoelasticity was measured by CUTOMETOR (CUTOMETER SEM575; Courage + Khazaka) before and after 4-week application. The relative value of the viscoelasticity on the back of the right hand based on that on the back of the left hand after 4-week application was calculated, and the statistical significance test was performed by the paired t-test.

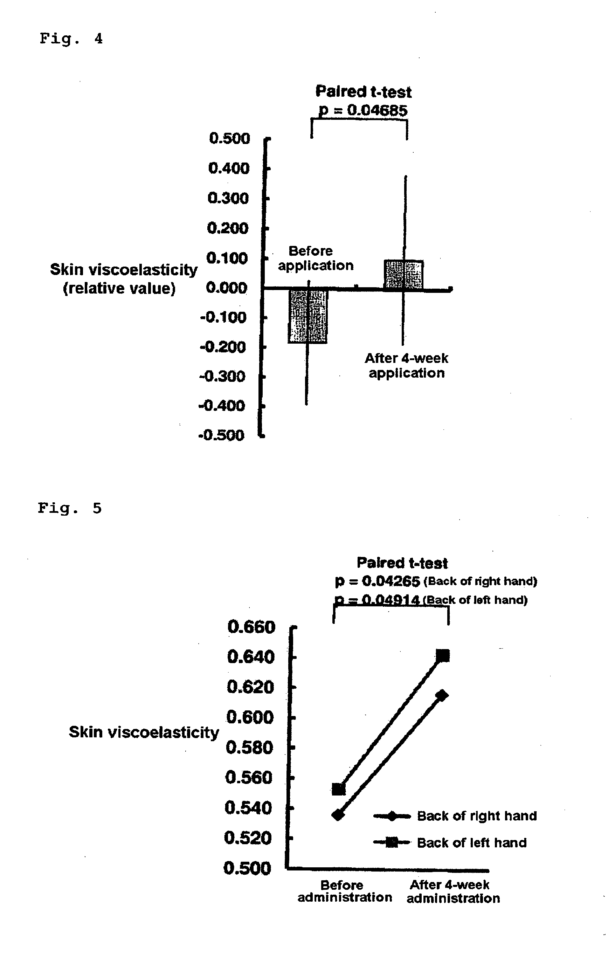

The results are shown in Fig. 4. The 4-week application of the 0.5% PI-PS containing phospholipid emulsified liquid onto the back of a hand significantly increased skin viscoelasticity.

At the same time, the skin moisture content and transepidermal water loss were also measured using CORNEOMETER CM 825 and AS-TW2, respectively. There was no marked difference between the measured values before and after the 4-week application of the 0.5% PI-PS containing phospholipid emulsified liquid.

### <Example 6>

### Skin monitor test using oral preparations in humans

Each of subjects in their 30s to 40s (3 males and 2 females) was instructed to orally take granules containing 400 mg of the PI-PS containing phospholipid prepared in Preparation Example 1, once a day, i.e. every morning, for 4 weeks. The skin viscoelasticity was measured in the same manner as in Example 5 before and after 4-week oral administration, and the significance test was performed.

The results are shown in Fig. 5. The 4-week oral administration of the PI-PS containing phospholipid significantly increased skin viscoelasticity.

At the same time, the skin moisture content and transepidermal water loss were also measured using CORNEOMETER CM 825 and AS-TW2, respectively. There was no marked difference between the measured values before and after the 4-week oral administration.

### Formulation Examples

**Table 1**

| Drink (milk drink) | |
|---|---|
| Ingredients | wt % |
| PI-PS containing PL mixture | 0.2 |
| milk | 99.8 |

| | |
|---|---|
| PL: phospholipid (the same shall apply hereinafter) | |

Milk was mixed with a PI-PS containing phospholipid mixture obtained in the same manner as in Preparation Example 1, and the mixture was stirred with TK homomixer at 5,000 rpm at room temperature for 2 min. The emulsified mixture was heated at 100°C for 10 minutes.

**Table 2**

| Drink (soy milk drink) | |
|---|---|
| Ingredients | wt % |
| PI-PS containing PL mixture | 0.2 |
| Xylo-oligosaccharide | 0.4 |
| Black sesame paste | 0.25 |
| Soy milk | 99.15 |

Soy milk was mixed with the above-mentioned ingredients, and the mixture was stirred with TK homomixer at 5,000 rpm at room temperature for 2 min. The emulsified mixture was heated at 100°C for 10 minutes. The PI-PS containing phospholipid mixture used was prepared in the same manner as in Preparation Example 1.

**Table 3**

| Drink (yogurt) | |
|---|---|
| Ingredients | wt % |
| PI-PS containing PL mixture | 0.6 |
| yogurt (drink type) | 99.3 |
| Vitamin E | 0.1 |

All the above-mentioned ingredients were mixed and stirred at room temperature, and thereby a drink was prepared as a uniform solution. The PI-PS containing phospholipid mixture used was prepared in the same manner as in Preparation Example 1.

**Table 4**

| Tablet | |
|---|---|
| Ingredients | wt % |
| PI-PS containing PL mixture | 20 |
| Lactose | 65 |
| Potato starch | 12 |
| Polyvinyl alcohol | 1.5 |
| Magnesium stearate | 1.5 |

A PI-PS containing phospholipid mixture, lactose and potato starch were mixed uniformly. After addition of an aqueous solution of polyvinyl alcohol, the mixture was granulated by the wet granulation method. The resulting granules were dried, magnesium stearate was mixed therewith, and the mixture was compressed into tablets each weighing 300 mg. The PI-PS containing phospholipid mixture used was prepared in the same manner as in Preparation Example 1.

### INDUSTRIAL APPLICABILITY

The glycerophospholipids phosphatidylserine and phosphatidylinositol increase the level of hyaluronan in the living body. A glycerophospholipid comprising phosphatidylserine and phosphatidylinositol improves skin elasticity. Therefore, these glycerophospholipids can be preferably used as an active ingredient of pharmaceuticals, cosmetics, foods with health claims, etc.

## Claims

1. A non-therapeutic method for increasing the level of hyaluronan in the skin, said method comprising administering, to a human, an effective amount of phosphatidylserine and phosphatidylinositol.

2. The method according to Claim 1, wherein the weight ratio of phosphatidylserine (PS) to phosphatidylinositol (PI) (PS:PI) administered to the human is 0.5 to 20:1 in terms of dry weight.

3. The method according to Claim 1 or 2, wherein the phosphatidylserine and phosphatidylinositol are externally administered to the skin of the human.

4. The method according to Claim 1 or 2, wherein the phosphatidylserine and phosphatidylinositol are orally administered to the human.

5. Phosphatidylserine and phosphatidylinositol for use in the treatment of a disease wherein hyaluronan level is decreased.

6. The phosphatidylserine and phosphatidylinositol according to Claim 5, wherein the weight ratio of phosphatidylserine (PS) to phosphatidylinositol (PI) (PS:PI) is 0.5 to 20:1 in terms of dry weight.

7. The phosphatidylserine and phosphatidylinositol according to Claim 5 or 6, which is for external use.

8. The phosphatidylserine and phosphatidylinositol according to Claim 5 or 6, which is for oral use.

9. The phosphatidylserine and phosphatidylinositol according to any of claims 5-8, wherein the disease wherein hyaluronan level is decreased involves deficiency of hyaluronan synthase or decreased expression thereof.

10. A non-therapeutic method for improving skin elasticity which comprises orally administering an effective amount of phosphatidylserine and phosphatidylinositol to a human.

11. The method according to Claim 10, wherein the weight ratio of phosphatidylserine (PS) to phosphatidylinositol (PI) (PS:PI) is 0.5 to 20:1 in terms of dry weight.

## Patentansprüche

1. Nicht-therapeutisches Verfahren zur Erhöhung des Hyaluronanspiegels in der Haut, wobei das Verfahren das Verabreichen einer wirksamen Menge Phosphatidylserin und Phosphatidylinositol an einen Menschen umfasst.

2. Verfahren nach Anspruch 1, wobei das Gewichtsverhältnis von Phosphatidylserin (PS) zu Phosphatidylinositol (PI) (PS:PI), das an den Menschen verabreicht wird, 0,5 bis 20 : 1, hinsichtlich des Trockengewichts, beträgt.

3. Verfahren nach Anspruch 1 oder 2, wobei das Phosphatidylserin und Phosphatidylinositol äußerlich der Haut des Menschen verabreicht werden.

4. Verfahren nach Anspruch 1 oder 2, wobei das Phosphatidylserin und Phosphatidylinositol oral an den Menschen verabreicht werden.

5. Phosphatidylserin und Phosphatidylinositol zur Verwendung bei der Behandlung einer Erkrankung, bei der der Hyaluronanspiegel gesenkt ist.

6. Phosphatidylserin und Phosphatidylinositol nach Anspruch 5, wobei das Gewichtsverhältnis von Phosphatidylserin (PS) zu Phosphatidylinositol (PI) (PS:PI) 0,5 bis 20 : 1, hinsichtlich des Trockengewichts, beträgt.

7. Phosphatidylserin und Phosphatidylinositol nach Anspruch 5 oder 6, welche für die äußerliche Verwendung vorgesehen sind.

8. Phosphatidylserin und Phosphatidylinositol nach Anspruch 5 oder 6, welche für die orale Verwendung vorgesehen sind.

9. Phosphatidylserin und Phosphatidylinositol nach einem der Ansprüche 5 - 8, wobei die Erkrankung, bei der der Hyaluronanspiegel gesenkt ist, einen Fehler bei der Hyaluronansynthase oder eine verringerte Expression davon involviert.

10. Nicht-therapeutisches Verfahren zur Verbesserung der Hautelastizität, welches das orale Verabreichen einer wirksamen Menge Phosphatidylserin und Phosphatidylinositol an einen Menschen umfasst.

11. Verfahren nach Anspruch 10, wobei das Gewichtsverhältnis von Phosphatidylserin (PS) zu Phosphatidylinositol (PI) (PS:PI) 0,5 bis 20 : 1, hinsichtlich des Trockengewichts, beträgt.

## Revendications

1. Procédé non thérapeutique pour augmenter le taux de hyaluronane dans la peau, ledit procédé comprenant l'administration, à un humain, d'une quantité efficace de phosphatidylsérine et de phosphatidylinositol.

2. Procédé selon la Revendication 1, dans lequel le rapport pondéral de la phosphatidylsérine (PS) au phosphatidylinositol (PI) (PS/PI) administrés à l'humain est de 0,5 à 20/1 en termes de poids sec.

3. Procédé selon la Revendication 1 ou 2, dans lequel la phosphatidylsérine et le phosphatidylinositol sont administrés par voie externe sur la peau de l'humain.

4. Procédé selon la Revendication 1 ou 2, dans lequel la phosphatidylsérine et le phosphatidylinositol sont administrés par voie orale à l'humain.

5. Phosphatidylsérine et phosphatidylinositol pour une utilisation dans le traitement d'une maladie dans laquelle le taux d'hyaluronane est réduit.

6. Phosphatidylsérine et phosphatidylinositol selon la Revendication 5, dans lesquels le rapport pondéral de la phosphatidylsérine (PS) au phosphatidylinositol (PI) (PS/PI) est de 0,5 à 20/1 en termes de poids sec.

7. Phosphatidylsérine et phosphatidylinositol selon la Revendication 5 ou 6, qui est pour un usage externe.

8. Phosphatidylsérine et phosphatidylinositol selon la Revendication 5 ou 6, qui est pour un usage oral.

9. Phosphatidylsérine et phosphatidylinositol selon l'une quelconque des revendications 5 à 8, la maladie dans laquelle le taux d'hyaluronane est réduit étant liée à un déficit en hyaluronane synthase ou à une expression réduite de celle-ci.

10. Procédé non thérapeutique pour améliorer l'élasticité de la peau qui comprend l'administration orale d'une quantité efficace de phosphatidylsérine et de phosphatidylinositol à un humain.

11. Procédé selon la Revendication 10, dans lequel le rapport pondéral de la phosphatidylsérine (PS) au phosphatidylinositol (PI) (PS/PI) est de 0,5 à 20/1 en termes de poids sec.


### SEQUENCE LISTING

<110> Nagase ChemteX Corporation
<120> Agent for increasing hyaluronic acid
<130> N17F3028
<150> JP2008-053757
   <151> 2008-03-04
<160> 4
<170> PatentIn version 3.1
<210> 1
   <211> 20
   <212> DNA
   <213> Artificial sequence
<220>
   <223> PCR primer
<400> 1
   gcctcatctg tggagatggt 20
<210> 2
   <211> 20
   <212> DNA
   <213> Artificial sequence
<220>
   <223> PCR primer
<400> 2
   atgcactgaa cacacccaaa 20
<210> 3
   <211> 20
   <212> DNA
   <213> Artificial sequence
<220>
   <223> PCR primer
<400> 3
   ggacttcgag caagagatgg 20
<210> 4
   <211> 20
   <212> DNA
   <213> Artificial sequence
<220>
   <223> PCR primer
<400> 4
   agcactgtgt tggcgtacag 20